# FASCICULE DE BREVET EUROPEEN

(11) **EP 0 470 912 B1**
(45) Date de publication et mention de la délivrance du brevet: **17.05.1995**
(21) Numéro de dépôt: 91420213.0
(22) Date de dépôt: 27.06.1991
(51) Int. Cl.: A61F 2/46, A61F 2/34

(54) **Ensemble prothétique comprenant notamment une cupule et un appareil impacteur**
Protheseneinrichtung bestehend aus Hüftpfanne und Einschlaggerät
Prosthetic assembly comprising a hip cup and an impact apparatus

(30) Priorité: 29.06.1990 FR 9008648
(43) Date de publication de la demande: 12.02.1992
(73) Titulaire: MEDINOV SA, F-42312 Roanne Cedex (FR)
(72) Inventeur: Chambaud, Denis, F-42153 Riorges (FR); Frick, Michel, 26 Chemin Joseph Aiguier 13009 Marseille (FR); Grobost, Jérôme, F-72000 Le Mans (FR); Levai, Jean-Paul,, F-63000 Clermont Ferrand (FR); Lucet, Alain, F-21850 Apollinaire (FR); Roux, Jean-Pierre,, F-75006 Paris (FR)
(74) Mandataire: Dupuis, François

(56) Documents cités:
- EP-A- 0 190 982
- EP-A- 0 214 885
- DE-U- 8 530 793
- FR-A- 2 548 012
- US-A- 4 662 891

## Description

Plus particulièrement l'invention concerne un ensemble prothétique pour implant cotyloïdien du type de ceux présentant une cupule ou anneau métallique recevant un noyau, notamment en polyéthylène.

D'une manière parfaitement connue, de tels implants sont destinés à remplacer la cavité cotyloïde de l'os iliaque, dans laquelle s'articule la tête du fémur. La cupule peut être fixée dans la cavité cotyloïde au moyen de différents agencements, avec ou sans ciment.

Le problème que se propose de résoudre l'invention est d'assurer l'impaction de l'anneau ou cupule dans la cavité cotyloïde, d'une manière simple, efficace et rationnelle en se fixant pour objectif de remédier aux inconvénients des techniques généralement utilisées à ce jour.

Selon l'état de la technique connue, (voir US-A-4 662 891), après avoir convenablement préparé la cavité cotyloïde au moyen notamment d'alésoirs de différents types, on assujettit la cupule à un appareil impacteur pour sa mise en place. Généralement, la plupart des appareils impacteurs utilisés sont conformés pour être vissés dans un trou taraudé exécuté dans le fond de la cupule. Pour l'essentiel, ce type d'appareil est exécuté à partir d'une tige filetée munie, à son extrémité d'un élément faisant office de poignée. Après avoir vissée la cupule à la tige filetée pour leur accouplement relatif, on tape à l'aide d'un marteau sur la poignée pour l'impaction de ladite cupule dans la cavité cotyloïde.

On conçoit que cette manière de procéder n'est pas rationnelle et est anti-mécanique. En effet, il n'est pas normal de taper sur un filetage. Des risques de dévissage peuvent donc apparaître. En outre, compte tenu de la conformation de ce type d'appareil impacteur, il n'est pas aisé d'orienter convenablement la cupule.

Un autre inconvénient apparaît également eu égard à la conception de la cupule en tant que telle résultant de l'usinage du trou taraudé dans le fond de la cavité hémisphérique nécessaire à l'accouplement de l'appareil impacteur. En effet, de l'os va se former au niveau de ce trou, entraînant, dans ces conditions, de réels problèmes lorsqu'il est nécessaire d'enlever l'implant cotyloïdien, notamment la cupule.

Par le Brevet US 4.662.891 (NOILES), on connaît un appareil permettant l'impaction d'une cupule ou anneau et susceptible de remédier aux inconvénients précités. Cependant, la solution enseignée par ce Brevet n'est pas satisfaisante et ne permet pas, d'une manière simple et efficace, d'impacter l'anneau. Les agencements de l'appareil et de l'anneau nécessitent de la part du praticien des manipulations qui peuvent s'avérer difficiles et par conséquent susceptibles de nuire à une bonne impaction de l'anneau.

L'invention s'est donc fixée pour but de remédier à ces inconvénients en apportant une solution au niveau de la cupule et de l'appareil impacteur, en ayant pour but de résoudre le problème posé d'assurer une impaction dans de très bonnes conditions anatomiques avec possibilité d'orientation contrôlée de la cupule dans la cavité cotyloïde, et de faciliter le démontage.

Pour résoudre un tel problème, l'ensemble prothétique selon l'invention comprend une cupule ou anneau et un appareil impacteur équipé d'agencements aptes à coopérer avec des agencements internes répartis sur la circonférence de la cupule, avec capacité d'indexation et de blocage angulaire, les agencements de l'anneau étant constitués par une entaille très sensiblement semi-circulaire, formée à partir du bord périphérique externe de la cavité de la cupule, le fond de ladite entaille étant prolongé sur au moins un côté par une encoche.

Il apparaît donc que les agencements de la cupule permettant son accouplement à l'appareil impacteur, sont convenablement répartis sur la périphérie interne de la cupule et non plus situés au fond de cette dernière. Compte tenu de la conception particulière de la cupule, les agencements de la partie de l'appareil impacteur sont formés en débordement de la péiphérie d'un disque dont le diamètre correspond au diamètre interne de la cavité de la cupule, le disque étant porté par une tête de liaison présentant des moyens d'accouplement, d'une manière démontable, sur l'appareil impacteur.

Suivant une autre caractéristique, les agencements du disque sont constitués par des ergots faisant office de galet et aptes à être engagés successivement par un mouvement linéaire et un mouvement angulaire dans l'entaille et l'encoche du système à baïonnette.

Un autre problème important que se propose de résoudre l'invention, est de rendre solidaires, de manière temporaire, la cupule et l'appareil impacteur, après accouplement de ces derniers afin de pouvoir orienter à volonté dans différents plans et selon différentes orientations angulaires, ladite cupule au moyen de l'appareil impacteur.

Un tel problème est résolu en ce que l'appareil présente un fourreau tubulaire faisant office de manche préhenseur, ledit fourreau étant équipé d'organes aptes à assurer, en combinaison avec une partie de la tête de liaison, le blocage et l'accouplement temporaire du disque et de la cupule en position d'indexation de leurs agencements respectifs.

Dans une forme de réalisation, les organes de blocage sont constitués par un élément apte à coopérer en appui dans le fond de la cavité de la cupule et assujetti à des moyens de commande que présente le manche et/ou le disque pour provoquer, en position de butée dudit élément et d'indexation des agencements du disque et de la cupule, le déplacement coaxial du disque pour assurer en combinaison avec lesdits agencements, l'accouplement et le blocage angulaire de l'ensemble de l'appareil impacteur et de la cupule.

Un autre problème que se propose de résoudre l'invention est d'assurer l'accouplement, de manière démontable, de la partie active de l'appareil impacteur sur le manche de l'appareil en vue de l'interchangeabilité de ladite partie en fonction des différentes tailles possibles de cupules.

Un tel problème est résolu en ce que les moyens d'accouplement sont constitués par un jeu de billes escamotables aptes à coopérer avec une gorge que présente une portée circulaire formée d'une équerre recevant le disque.

Suivant une autre caractéristique, pour résoudre le problème posé de travailler dans des conditions opératoires efficaces et rationnelles, le disque présente des ouvertures pour donner un libre accès visuel au fond de la cupule.

L'invention est exposée ci-après plus en détail, à l'aide les dessins annexés dans lesquels :
La figure 1 est une vue en coupe illustrant l'appareil impacteur selon l'invention composé d'un manche préhenseur et de commande assemblé à un moyen de liaison avec une cupule ou anneau.
La figure 2 est une vue en coupe partielle du moyen de liaison seul.
La figure 3 est une vue de dessous correspondant à la figure 2;
La figure 4 est une vue en plan illustrant une cupule équipée des agencements selon l'invention.
La figure 5 est une vue partielle en perspective et à plus grande échelle de la cupule selon la figure 4.
La figure 6 est une vue en coupe partielle montrant le moyen de liaison appliqué sur la cupule et représenté en position déverrouillée.
La figure 7 est une vue en plan et en coupe correspondant à la figure 6.
La figure 8 est une vue en coupe partielle semblable à la figure 6 avec le moyen de liaison a représenté en position verrouillée.
La figure 9 est une vue en plan et en coupe correspondant à la figure 8.

L'ensemble prothétique selon l'invention comprend, d'une manière connue, une cupule (1) et un appareil impacteur désigné dans son ensemble par (A).

Selon l'invention, la cavité hémisphérique (1a) de la cupule présente des agencements internes périphériques (1b) convenablement réparties sur la circonférence de ladite cavité. Ces agencements sont conformés pour recevoir, d'une manière démontable, avec capacité d'indexation et de blocage angulaire, des agencements complémentaires (2a) que présente une partie de l'appareil impacteur sous forme notamment d'une tête de liaison désignée dans son ensemble par (T). Comme il sera indiqué dans la suite de la description, cette tête de liaison (T) est conformée pour être adaptée d'une manière démontable sur un manche (M) de préhension que présente l'appareil impacteur.

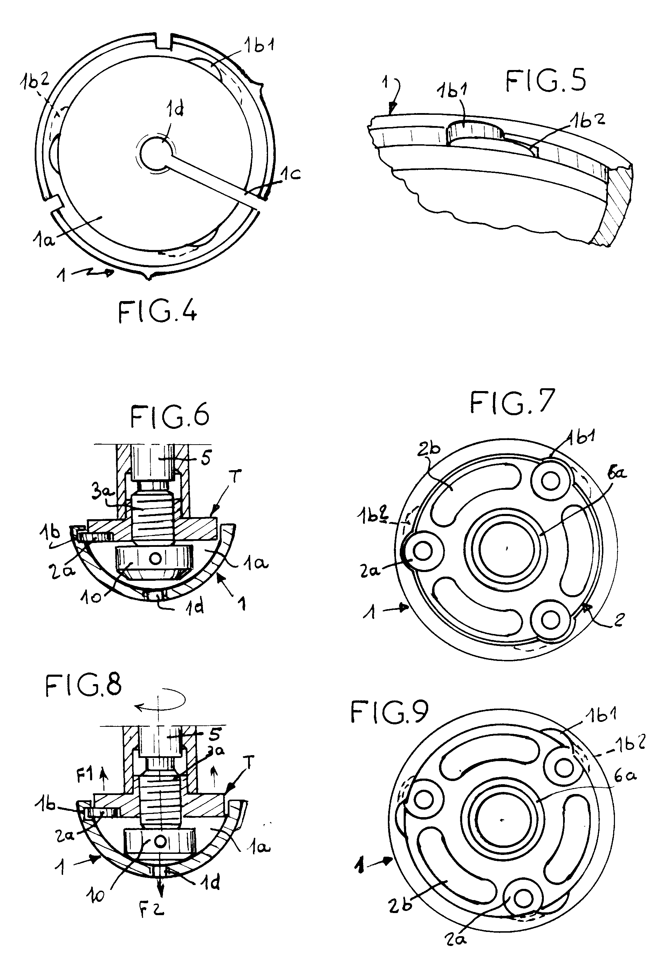

Les agencements (1b) de la cupule sont constitués par un système à baïonnette. Dans ce but, chacun des agencements est constitué par une entaille très sensiblement circulaire (1b1) formée à partir du bord périphérique externe de la cavité. Le fond de l'entaille (1b1) est prolongé sur au moins un côté par une encoche (1b2) (figures 4 et 5).

A noter que les différents systèmes à baïonnette (1b) peuvent être disposés symétriquement par rapport au centre de la cavité hémisphérique, en étant régulièrement décalés angulairement. On n'exclut cependant pas une disposition asymétrique de ces agencements (1b).

En ce qui concerne les agencements (2a) de la tête de liaison (T), ces derniers sont formés en débordement de la périphérie d'un disque (2), dont le diamètre correspond très sensiblement au diamètre interne de la cavité hémisphérique (1a) pour mettre en coopération les agencements (1b - 2a). Les agencements (2a) sont constitués par des ergots faisant office de galets, et aptes à être engagés successivement par un mouvement linéaire et angulaire dans l'entaille (1b1 - 1b2) de chacun des systèmes à baïonnette (1b). Une partie des ergots (2a) apparaît en débordement de la circonférence du disque (2).

Dans la forme d'exécution illustrée dans la figure des dessins, qui constitue un exemple de réalisation préféré, quine saurait cependant être considéré comme limitatif, le disque (2) de la tête de liaison est engagé dans une portée filetée (3a) que présente une queue (3). L'ensemble de la tête de liaison est conformé pour être accouplé d'une manière démontable au manche de préhension (M).

Dans ce but, la queue (3) présente une gorge circulaire (3b) destinée à recevoir un jeu de billes (4) porté par un axe (5) engagé à libre coulissement dans un fourreau (6) constituant l'essentiel du manche de préhension (M). L'extrémité (6a) du fourreau présente un chambrage interne (6b) pour le logement des billes (4) correspondant à une position de désaccouplement comme il sera indiqué ci-après.

L'extrémité du manche de préhension, à l'opposé de son extrémité d'accouplement avec la tête de liaison, reçoit un embout épaulé (7) monté avec capacité de coulissement le long de l'axe (5) à l'encontre d'un ressort de rappel (8) en butée contre un bouton (11) fixé à l'extrémité dudit axe (5) et permettant d'agir en rotation sur ce dernier. Une poignée latérale (9) est fixée angulairement sur une partie du fourreau (6) pour permettre une parfaite préhension de l'ensemble de l'appareil et faciliter son orientation selon différentes positons angulaires en combinaison avec le manche constitué par ledit fourreau.(6).

Selon une autre caractéristique importante de l'invention, l'extrémité de la queue (3) débordant du disque (2) reçoit un élément (10) sous forme d'un patin profilé. Ce patin (10) est apte à prendre appui dans le fond de la cavité hémisphérique pour assurer le blocage et l'accouplement temporaire du disque (2) et de la cupule (1) en position d'indexation de leurs agencements respectifs (2a - 1b) pour assurer une solidarisation temporaire de la tête de liaison recevant le disque et de la cupule et conséquemment leur blocage angulaire relatif.

Ce blocage angulaire s'effectue par un déplacement coaxial selon deux sens opposés (F1 - F2) (figure 8) du disque (2) et du patin d'appui (10).

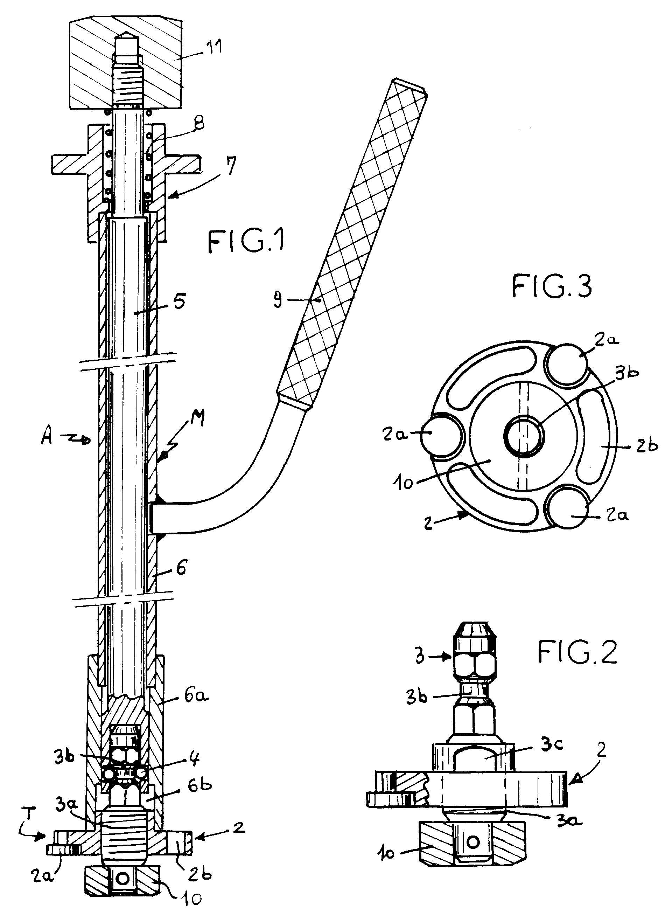

Il convient maintenant d'analyser le fonctionnement de l'ensemble prothétique selon l'invention, en se référant plus particulièrement aux figures 1, 6, 7, 8 et 9.

Il convient en premier lieu de procéder à l'accouplement des deux parties de l'appareil impacteur, à savoir le manche de préhension (6) et la tête de liaison (T) avec la cupule (1). Pour assurer l'accouplement de la tête de liaison (T), en bout du manche, notamment de l'axe (5), on agit de manière simultanée, sur l'embout épaulé (7) et le bouton d'extrémité (11) à l'encontre du ressort de rappel (8), afin que l'extrémité de l'axe (5) portant les billes (4) se déplace par rapport à l'extrémité (6a) du fourreau (6). Dans cette position, les billes (4) s'écartent radialement et se loge dans le chambrage (6b) de l'extrémité du fourreau. Il est alors possible de procéder à l'engagement de l'extrémité de l'ensemble du manche sur la queue (3) de la tête de liaison. En fin de course, après relachement du bouton (11) et de l'embout (7), le fond du chambrage (6b) repousse les billes en vue de leur engagement dans la gorge circulaire (3b) de la queue (3). L'extrémité (6a) du fourreau présente intérieurement, au moins un méplat apte à coopérer avec une portée (3c) de section complémentaire formée en débordement du disque (2). Les deux parties de l'appareil impacteur, à savoir la tête de liaison (T) et le manche de préhension (M) sont ainsi liés angulairement et en translation pour se comporter comme un organe de manoeuvre unique.

On se réfère maintenant plus particulièrement aux figures 6 à 9, pour décrire la liaison et l'accouplement de l'ensemble de l'appareil impacteur tel que défini, avec une cupule en vue de sa mise en place dans la cavité cotyloïde de l'os illiaque.

La cupule (1) peut être saisie manuellement dans son conditionnement stérile, disposée dans la cavité cotyloïde, puis accouplée à l'appareil impacteur. Ou bien, la cupule est saisie directement par l'appareil impacteur dans son conditionnement.

Pour assurer la liaison entre la cupule et l'ensemble de l'appareil, il suffit d'engager linéairement, les ergots (2a) du disque (2) dans les dans les entailles (1b1) jusqu'en position de butée (figures 6 et 7). L'appareil impacteur est alors pivoté angulairement, notamment au moyen de sa poignée latérale (9) pour provoquer d'une manière concomittante, le déplacement angulaire du disque (2) pour l'engagement des ergots (2a), dans les encoches latérales (1b2) (figure 9). Dans cette position, l'ensemble prothétique est verrouillé mais n'est pas encore bloqué angulairement. Pour assurer le blocage angulaire le l'ensemble de l'appareil impacteur et de la cupule (1), c'est-à-dire assurer une solidarisation temporaire de ces deux éléments, on agit manuellement en rotation sur le bouton d'extrémité (11) de l'axe (5). Compte-tenu de la liaison angulaire de l'extrémité de l'axe (5) avec la queue (3) au moyen des billes (4), la portée filetée (3 ) se visse dans le disque (2), provoquant ainsi le déplacement linéaire concomittant du patin d'appui (10) jusqu'en position de butée dans le fond de la cavité hémisphérique de la cupule. Lorsque l'on continue d'agir sur le bouton, on provoque par réaction (flèche F1, F2, - Figure 8), le déplacement du disque (2) jusqu'à ce que les ergots (2a) viennent en appui sur la face supérieure des encoches (1b2), assurant ainsi, sous un effet de coincement, le blocage angulaire de l'ensemble de l'appareil impacteur et de la cupule.

Il est donc possible de manoeuvrer selon différentes orientations angulaires, l'ensemble prothétique pour permettre l'orientation contrôlée de la cupule en vue d'une parfaite impaction dans la cavité cotyloïde. Pour procéder au retrait de l'ensemble de l'appareil impacteur, il suffit d'agir en sens inverse sur le bouton (11) pour supprimer la pression de blocage, afin de ramener le disque (2) et ses ergots (2a) en position de verrouillage (figure 6).

L'ensemble prothétique tel que défini, peut s'appliquer à tout type de cupule à la seule condition que cette dernière présente les mêmes caractéristiques ou des caractéristiques équivalentes au niveau des agencements d'accouplement avec l'appareil impacteur. Dans l'exemple illustré, la cupule (1) est du type pré-orientée en présentant une fente radiale (1c) en communication avec un trou central (1d) pratiqué dans le fond de la cavité hémisphérique. Pour assurer une fixation primaire de la cupule, cette dernière peut présenter en débordement de sa périphérie externe, des aillettes aptes à être impactées au niveau des deux cornes du cotyle. Par ailleurs, la périphérie externe de la cupule peut présenter tout type d'agencement tels que des bossages convenablement disposés pour créer un appui tripode, ainsi que tout traitement de surface.

Les avantages ressortent bien de la description, en particulier on souligne et on rappelle la facilité d'accouplement de l'ensemble de l'appareil impacteur en combinaison avec les agencements périphériques de la cupule, permettant ainsi de l'orienter selon différents plans, d'une manière particulièrement sûre et efficace.

On souligne également que le disque (2) présente dans son épaisseur, de larges ouvertures (2b) pour donner un libre accès visuel au fond de la cupule, permettant ainsi d'agir en toute sécurité pour l'orientation et l'impaction de la cupule.

## Revendications

1. Ensemble prothétique comprenant une cupule ou anneau (1) et un appareil impacteur (2) équipé d'agencements (2a) aptes à coopérer avec des agencements internes (1b) répartis sur la circonférence de la cupule (1), avec capacité d'indexation et de blocage angulaire, les agencements (1b) étant constitués par une entaille (1b1) très sensiblement semi-circulaire, formée à partir du bord périphérique externe de la cavité (1a) de la cupule, le fond de ladite entaille (1b1) étant prolongé sur au moins un côté par une encoche (1b2), les agencements (2a) de la partie de l'appareil impacteur sont formés en débordement de la périphérie d'un disque (2) dont le diamètre correspond au diamètre interne de la cavité de la cupule, caractérisé en ce que le disque est porté par une tête de liaison présentant des moyens d'accouplement (4-3b), d'une manière démontable, sur l'appareil impacteur, qui présente un fourreau tubulaire (6) faisant office de manche préhenseur, ledit fourreau étant équipé d'organes (11-10) aptes à assurer, en combinaison avec une partie de la tête de laision, le blocage et l'accouplement temporaire du disque et de la cupule en position d'indexation de leurs engagements respectifs (2a) (1b).

2. Ensemble selon la revendication 1, caractérisé en ce que les organes de blocage sont constitués par un élément (10) apte à coopérer en appui dans le fond de la cavité de la cupule et assujetti à des moyens de commande (11) que présente le manche et/ou le disque pour provoquer, en position de butée dudit élément (10) et d'indexation des agencements (2a-1b) du disque et de la cupule, le déplacement coaxial du disque (2) pour assurer en combinaison avec lesdits agencements, l'accouplement et le blocage angulaire de l'ensemble de l'appareil impacteur et de la cupule.

3. Ensemble selon la revendication 1, caractérisé en ce que les agencements (2a) du disque sont constitués par des ergots faisant office de galet et aptes à être engagés successivement par un mouvement linéaire et un mouvement angulaire, dans l'entaille (1b1) et l'encoche (1b2) du système à baïonnette.

4. Ensemble selon la revendication 1, caractérisé en ce que les moyens d'accouplement sont constitués par un jeu de billes escamotables (4) aptes à coopérer avec une gorge que présente une portée circulaire (3b) formée d'une queue (3) recevant le disque.

5. Ensemble selon la revendication 1, caractérisé en ce que le disque (2) présente des ouvertures (2b) pour donner un libre accès visuel au fond de la cupule.

## Claims

1. Prosthesis assembly comprising a cup or ring (1) and an impacting device (2) provided with features (2a) suitable for cooperating with internal features (1b) distributed over the circumference of cup (1) with an indexing and angular locking facility, the features (1b) consisting of a substantially semi-circular notch (1b1) formed on the outer peripheral edge of the cavity (1a) of the cup, the bottom of said notch (1b1) being extended on at least one side by a recess (1b2), the features (2a) of the impacting device part are formed so that they project beyond the periphery of a disc (2) of which the diameter matches the inside diameter of the cavity of the cup, characterised in that the disc is supported by a connecting head having means of coupling (4-3b), in a way which makes it removable, to the impacting device which has a tubular sleeve (6) acting as a gripping handle, said sleeve being fitted with devices (11-10) capable of ensuring, in combination with a part of the connecting head, locking and temporary coupling of the disc and the cup in the indexed position of their respective mating parts (2a) (1b).

2. Assembly as claimed in claim 1, characterised in that the locking devices consist of an element (10) capable of cooperating with the cavity of the cup by pressing into the bottom of the latter, and controlled by means of control (11) that the handle and/or the disc have so as to cause, in the limit-stop position of said element (10) and with features (2a-1b) of the disc and the cup indexed, coaxial displacement of disc (2) in order to ensure, in combination with said features, coupling and angular locking of the impacting device and cup assembly.

3. Assembly as claimed in claim 1, characterised in that the features (2a) of the disc consist of studs that act as rollers and are capable of being successively engaged, by a linear movement and an angular movement, in recess (1b1) and notch (1b2) of the bayonet system.

4. Assembly as claimed in claim 1, characterised in that the means of coupling consist of a set of retractable balls (4) capable of cooperating with a groove which a circular bearing surface (3b) has that consists of a shank (3) that accommodates the disc.

5. Assembly as claimed in claim 1, characterised in that the disc (2) has openings (2b) in order to allow free visual access to the bottom of the cup.

## Patentansprüche

1. Prothesenkonstruktion mit einer Gelenkpfanne bzw. Ring (1) und einem Anschlaggerät (2), das Vorkehrungen (2a) besitzt, die so ausgebildet sind, um mit auf dem Umfang der Pfanne (1) verteilten innenseitigen Vorkehrungen (1b) zusammenzuwirken, mit Möglichkeit zur Indexierung und Winkelblockierung, wobei die Vorkehrungen (1b) aus einem ziemlich genau halbkreisförmigen Einschnitt (1b1) bestehen, der aus dem äußeren Rand der Vertiefung (1a) der Pfanne gebildet ist, während der Boden dieses Einschnitts (1b1) auf mindestens einer Seite durch eine Einkerbung (1b2) verlängert wird und die Vorkehrungen (2a) des Anschlagteils über den Rand einer Scheibe (2) hinausragen, deren Durchmesser dem Innendurchmesser der Pfannenvertiefung entspricht, dadurch gekennzeichnet, daß die Scheibe durch einen Verbindungskopf getragen wird, der abnehmbare Vorrichtungen (4-3b) zum Ankoppeln an das Anschlaggerät besitzt, das eine rohrförmige Hülle (6) aufweist, die als Griff dient, wobei die Hülle mit Organen (11-10) versehen ist, die geeignet sind, im Zusammenspiel mit einem Teil des Verbindungskopfes für die Blockierung und zeitweise Ankoppelung der Scheibe und der Pfanne in der Indexstellung ihrer jeweiligen Eingriffe (2a) (1b) zu sorgen.

2. Konstruktion nach Anspruch 1, dadurch gekennzeichnet, daß die Sperrorgane aus einem Bauteil (10) bestehen, das geeignet ist, im Andruck mit dem Boden der Pfannenvertiefung zusammenzuwirken, und das von Steuervorrichtungen (11) des Griffes und/oder der Scheibe abhängt, um in der Anschlagstellung des Bauteils (10) und in der Indexstellung der Vorkehrungen (2a-1b) der Scheibe und der Pfanne eine koaxiale Verschiebung der Scheibe (2) zu bewirken und so im Zusammenspiel mit den besagten Vorkehrungen für die Ankoppelung und Winkelblockierung des gesamten Anschlaggeräts und der Pfanne zu sorgen.

3. Konstruktion nach Anspruch 1, dadurch gekennzeichnet, daß die Vorkehrungen (2a) der Scheibe aus Nocken bestehen, die als Rolle dienen und durch eine lineare und eine Winkelbewegung nacheinander in den Einschnitt (1b1) und in die Einkerbung (1b2) des Bajonettensystems eingeführt werden können.

4. Konstruktion nach Anspruch 1, dadurch gekennzeichnet, daß die Ankoppelungsvorrichtungen aus einem Satz versenkbarer Kugeln (4) bestehen, die geeignet sind, mit einer Auskehlung einer kreisförmigen Auflagefläche (3b) zusammenzuwirken, die aus einem die Scheibe aufnehmenden Endstück (3) gebildet ist.

5. Konstruktion nach Anspruch 1, dadurch gekennzeichnet, daß die Scheibe (2) Öffnungsschlitze (2b) für den freien Blick auf den Boden der Pfanne aufweist.
